(19) Europäisches Patentamt
European Patent Office
Office européen des brevets

(11) EP 1 589 955 B1

(12) **EUROPÄISCHE PATENTSCHRIFT**

(45) Veröffentlichungstag und Bekanntmachung des Hinweises auf die Patenterteilung:
**02.11.2006 Patentblatt 2006/44**

(21) Anmeldenummer: **04707525.4**

(22) Anmeldetag: **03.02.2004**

(51) Int Cl.:
***A61K 9/70*** *(2006.01)* ***A61K 31/4468*** *(2006.01)*

(86) Internationale Anmeldenummer:
**PCT/EP2004/000944**

(87) Internationale Veröffentlichungsnummer:
**WO 2004/069286 (19.08.2004 Gazette 2004/34)**

(54) **FÜR DIE WÄRMEANWENDUNG ZUR BESCHLEUNIGUNG DER PERMEATION VON WIRKSTOFFEN GEEIGNETES TRANSDERMALES THERAPEUTISCHES SYSTEM UND SEINE VERWENDUNG**

TRANSDERMAL THERAPEUTIC SYSTEM SUITABLE FOR HEAT APPLICATION FOR PROMOTING THE PERMEATION OF ACTIVE SUBSTANCES, AND THE USE THEREOF

SYSTEME THERAPEUTIQUE TRANSDERMIQUE APTE A UNE APPLICATION THERMIQUE VISANT A AUGMENTER LA PERMEATION DE SUBSTANCES ACTIVES, ET UTILISATION ASSOCIEE

(84) Benannte Vertragsstaaten:
**AT BE BG CH CY CZ DE DK EE ES FI FR GB GR HU IE IT LI LU MC NL PT RO SE SI SK TR**

(30) Priorität: **07.02.2003 DE 10304989**
**22.01.2004 DE 102004003224**

(43) Veröffentlichungstag der Anmeldung:
**02.11.2005 Patentblatt 2005/44**

(60) Teilanmeldung:
**06017854.8**

(73) Patentinhaber: **LTS LOHMANN Therapie-Systeme AG**
**56626 Andernach (DE)**

(72) Erfinder: **MÜLLER, Walter**
**56626 Andernach (DE)**

(74) Vertreter: **Schmidt, Werner et al**
**LTS Lohmann**
**Therapie-Systeme AG**
**Patentabteilung**
**Postfach 15 25**
**56605 Andernach (DE)**

(56) Entgegenhaltungen:
**WO-A-89/10108** **US-B1- 6 261 595**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Transdermale Therapeutische Systeme ( TTS ) sind mittlerweile eine gut eingeführte Arzneiform geworden, die wegen ihrer therapeutischen Vorteile auch von den Patienten gut akzeptiert wird. Aufgrund der Barrierewirkung der menschlichen Haut ist allerdings die transdermale Verabreichung von Wirkstoffen mit topischer und systemischer Wirkung auf Substanzen beschränkt, die auf Grund ihrer Wirksamkeit bei schon geringen Dosen und ihrer physikalisch-chemischen Eigenschaften auf einer akzeptablen Fläche in pharmakologisch ausreichender Menge durch die Haut permeieren können.

Es gab und gibt deshalb zahlreiche Anstrengungen die Barrierewirkung der Haut zu beeinflussen und dadurch das Spektrum der für die transdermale Therapie geeigneten Wirkstoffe zu erweitern. Ein erster Ansatz dieses Ziel zu erreichen war die Verwendung von speziellen Substanzen, den sogenannten Permeationsenhancern, die ebenfalls in die Haut eindiffundieren und die Struktur des Stratum Corneums, der Hauptdiffusionsbarriere, beeinflussen. Beispielhaft seien genannt Fettalkohole, Fettsäuren und Fettsäureester, Dimethylsulfoxid, Glycerinderivate, polyoxyethylierte Fettalkohole und Fettsäuren.

Neben diesem sogenannten chemischen Enhancement wurden auch physikalische Verfahren wie elektrischer Strom bei der sogenannten Iontophorese, Ultraschall bei der sogenannten Sonophorese und Wärme erfolgreich eingesetzt.

Das einfachste physikalische Mittel, um die Durchlässigkeit der Haut für chemische Substanzen zu steigern, ist die Einwirkung von Wärme.

Ein transdermales System, das sich diesen Effekt zu Nutze macht, ist in der US Patentschrift 4,898,592 beschrieben. Das dort beschriebene transdermale System enthält optional eine wärmeleitende Schicht, um die Körperwärme zur Erhöhung der Temperatur an der Applikationsstelle des TTS zu nutzen.

Während dieses System rein passiv arbeitet, ist in der US-Patentschrift 4,230,105 ein System beschrieben, das nach Aktivierung mit Wasser aktiv Wärme entwickelt und dadurch die Permeationsrate für Wirkstoffe steigert.

In der US-Patentschrift 5,919,479 ist ein Prinzip beschrieben, das die Anwendung von kontrollierter Wärme in Kombination mit der Anwendung von Lokalanesthetika offenbart. Die Wärme wird dabei durch die Reaktion von Luftsauerstoff mit auf Aktivkohle befindlichem pyrophorem Eisen in Gegenwart von Wasser erzeugt.

In der US-Patentschrift 6,261,595 wird beschrieben, wie wärmeerzeugende Vorrichtungen mit konventionellen transdermalen Systemen vorteilhaft kombiniert werden können. Es wird darauf hingewiesen, daß es besonders vorteilhaft ist, wenn das transdermale Systeme so beschaffen ist, daß das Wärme erzeugende bzw. -abgebende Element jederzeit angebracht, entfernt bzw. ausgetauscht werden kann, ohne das transdermale System zu schädigen. Die leichte Austausch- bzw. Entfernbarkeit ist speziell wichtig, wenn die Wirkstoffabgabe an die jeweiligen Bedürfnisse des Patienten angepasst werden soll. Dies ist z.B. besonders wünschenswert, wenn es sich bei dem Wirkstoff um ein Schmerzmittel handelt. Besonders bei nebenwirkungsreichen Schmerzmitteln wie Opiaten, Fentanyl und fentanylanalogen Substanzen sollte der Patient einerseits jeweils eine ausreichende Menge zugeführt bekommen, um beschwerdefrei zu sein, andererseits sollte wegen der Nebenwirkungen auch nicht mehr als die der jeweiligen Situation angemessene Menge verabreicht werden. Durch die Anwendung von Wärme kann bei auftretenden Schmerzen die Wirkstoffabgabe des Systems zeitweise erhöht und damit dem gestiegenen Bedarf angepasst werden.

In WO 89/10108 A wird ein TTS beschrieber, welches eine Rückschicht eine Matrixschicht auf der Basis von Polysiloxan und eine Schutzschicht enthält.

In keiner der obigen Patentschriften wird jedoch auf die besonderen Anforderungen an die Eigenschaften der Matrix, den wirkstoffhaltigen Teil von transdermalen Systemen, bei Anwendung von Wänne zur Erhöhung der Permeationsrate eingegangen.

Aufgabe dieser Erfindung ist es deshalb, Formulierungen für die wirkstoffhaltigen Teile eines Matrixsystems zu entwickeln, die besonders gut geeignet sind, die Wirkstoffabgabe für die ganze oder einen Teil der Applikationszeit durch die Anwendung von Wärme zu erhöhen.

Matrixsysteme bestehen im einfachsten Fall lediglich aus einer wirkstoffundurchlässigen Rückschicht, einer wirkstoffhaltigen und idealerweise selbstklebenden Matrixschicht und einer vor Gebrauch zu entfernenden Schutzschicht. Die wirkstoffhaltige Schicht besteht üblicherweise neben dem Wirkstoff zum größten Teil aus Polymeren auf der Basis von Polyacrylaten, Polyisobutylen, Polyisopren, Blockpolymeren aus Styrol-Polybutylen-Styrol, Polysiloxanen, Polyurethanen oder Hydrogelen. Speziell Matrices auf Basis von Polyisobutylen und Blockpolymeren enthalten zusätzliche Klebrigmacher auf der Basis von Kohlenwasserstoffharzen und/ oder Kolophoniumderivaten. Alle Matrices können zusätzliche Hilfsstoffe enthalten, die die Löslichkeit des Wirkstoffs beeinflussen bzw. Permeationsenhancer, die die Absorptionsrate aus dem System erhöhen.

Bei Matrixsystemen erfolgt die Steuerung der Wirkstoffaufnahme üblicherweise weniger durch das System, sondern zu einem großen Teil durch die Haut selbst. Allerdings gibt es die Möglichkeit, die Matrix hautseitig mit einer Steuermembran und die Membran gegebenenfalls mit einer Kleberschicht zur Verankerung des Systems auf der Haut zu versehen um dadurch eine bessere Systemkontrolle zu erreichen.

Die hautseitige Kleberschicht zur Verankerung auf der Haut kann ebenfalls mit Wirkstoff beladen werden. Dieser Anteil wird dann ohne Membrankontrolle abgegeben um z.B. ein mögliches Hautdepot für den Wirkstoff möglichst schnell zu sättigen.

Für die in-vivo Wirkstoffabgabe bei einfachen monolytischen Matrixsystemen ist neben den physikalisch-chemischen Eigenschaften des Wirkstoffs und der Anwesenheit und Wirksamkeit von chemischen Permeationsenhancern die thermodynamische Aktivität des Wirkstoffs der entscheidende, die Abgaberate bestimmende Parameter. In einem stabilen System ist die maximale thermodynamische Aktivität erreicht, wenn die Konzentration des Wirkstoffs der Sättigungskonzentration des Wirkstoffs in der Matrixformulierung entspricht. Eine weitere Erhöhung der Wirkstoffkonzentration führt zu keiner weiteren Steigerung der thermodynamischen Aktivität, wenn der die Sättigungslöslichkeit übersteigende Anteil des Wirkstoffs als reiner Wirkstoff, d.h. in Form von Kristallen, bzw. bei niedrig schmelzenden Wirkstoffen als Flüssigphase, in der Matrix vorliegt. Ist der Wirkstoff in einer die Sättigungskonzentration übersteigenden Menge in dem System gelöst, spricht man von übersättigten Systemen. Da der Wirkstoff in solchen Systemen dazu neigt, während der Lagerzeit zu rekristallisieren bzw. eine eigene Phase zu bilden, müssen solche Systeme als meta- bzw. instabil betrachtet werden.

Die oben geschilderten Zusammenhänge zeigen, daß es eine komplexe Wechselwirkung zwischen der aktuellen Wirkstoffkonzentration und den Löseeigenschaften der jeweiligen Matrixformulierung gibt. Diese Wechselwirkungen müssen speziell bei transdermalen Systemen, bei denen die Wirkstoffabgabe während der Applikationszeit oder zumindest bei einem Teil der Applikationszeit durch die Anwendung von Wärme gesteigert wird, berücksichtigt werden.

An ein solches System sind folgende Forderungen zu stellen:

1. Die thermodynamische Aktivität des Wirkstoffs sollte nach der Anwendung von externer Wärme unverändert sein

2. Die thermodynamische Aktivität des Wirkstoffs muss nach der Anwendung von Wärme schnell auf das Ausgangsniveau zurückgehen.

3. Das System muß so ausgelegt sein, dass auch bei Anwendung von Wärme und der damit verbundenen zeitweise erhöhten Abgaberate die Wirkstoffabgabe für die gesamte Applikationszeit auf dem gewünschten Niveau gewährleistet ist und sich das System nicht vorzeitig erschöpft..

Die Aufgabe wird erfindungsgemäß durch ein transdermales therapeutisches System gemäß Anspruch 1 der vorliegenden Anmeldung gelöst.

Gegenstand der Anmeldung ist daher ein für die Verabreichung mindestens eines pharmakologischen Wirkstoffes in einem oberhalb der Hauttemperatur liegenden Temperaturbereich geeignetes transdermales therapeutisches System (TTS), umfassend eine wirkstoffundurchlässige Rückschicht, mindestens eine wirkstoffhaltige Matrixschicht auf der Basis von mindestens einem Polysiloxan, eine abziehbare Schutzschicht, sowie ein internes und/oder externes wärmeabgebendes Element, dadurch gekennzeichnet, das

a) die wirkstoffhaltige Schicht ohne die Berücksichtigung des Wirkstoffes zu mindestens 80 Gew.-% aus Polysiloxan besteht,
b) der Wirkstoff zu höchstens 20 Gew.-% in gelöster Form vorliegt, und
c) der Wirkstoff in dem Polysiloxan eine Sättigungslöslichkeit von höchstens 5 Gew.-% besitzt.

In einer Ausführungsform enthält das erfindungsgemäße TTS, z. B. in Form einer Matrixschicht ein internes wärmeabgebendes Element.

Eine bevorzugte Ausführungsform der vorliegenden Erfindung ist ein TTS, das mit einem externen wärmeabgebenden Element, einem sogenannten Heatpack verbunden ist, welches entfernt bzw. ausgetauscht werden kann, ohne das transdermale System zu schädigen. Dieses Element kann gemäß einer weiteren Ausführungsform auch getrennt vom TTS, aber in einer gemeinsamen Verpackungseinheit vorliegen.

Gegenstand der vorliegenden Erfindung ist ferner die Verwendung des oben definierten TTS zur transdermalen Verabreichung von Wirkstoffen bei Temperaturen höher als die Hauttemperatur, dadurch gekennzeichnet, dass nach Entfernung der Schutzschicht die von der Rückschicht bedeckte Matrixschicht auf die Haut aufgebracht wird und entweder durch ein internes wärmeabgebendens Element oder durch Verbinden des TTS mit einem externen wärmeabgebenden Element (sogenanntes Heatpack) die Matrixschicht für eine bestimmte Zeit auf eine Temperatur, die höher als die Hauttemperatur ist, erwärmt wird. In einer bevorzugten Ausführungsform der Verwendung wird die Matrix auf eine Temperatur bis einschließlich 50 °C erwärmt.

Gegenstand der vorliegenden Erfindung ist ferner eine Verpackungseinheit, in welcher das TTS sowie das externe wärmeabgebende Element (Heatpack) getrennt von einander verpackt sind.

Erfindungsgemäße interne oder externe wärmeabgebende Elemente können solche sein, wie sie z. B. in den

US-Patentschriften 5,919,479 bzw. 6,261,595 beschrieben worden sind.

Polysiloxane (genauer: Polyorganosiloxane) sind in der Form von Lösungen oder als lösemittelfreie Zweikomponentensysteme erhältlich. Durch die Variation des organischen Teils der Polysiloxane gibt es eine Vielzahl von unterschiedlichen Polysiloxanen, wobei jedoch für transdermale Systeme bisher nur Polydimethylsiloxane eingesetzt werden.

Bei Zweikomponentensysteme hat das Polysiloxan ein relativ geringes Molekulargewicht und ist bei Raumtemperatur flüssig bis zähflüssig. Erst beim Zusammengeben der beiden Komponenten erfolgt eine Quervernetzung die je zu einem gebrauchsfähigen Produkt führt.

Für die Herstellung von transdermalen Systemen werden bevorzugt gelöste Polydimethylsiloxane gemäß folgender Strukturformel eingesetzt.

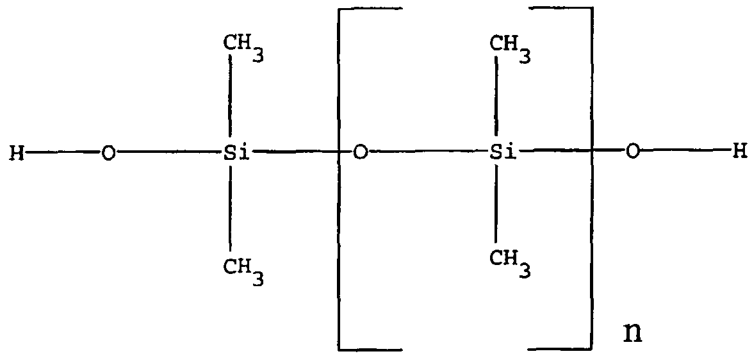

Durch die zusätzliche Verwendung von Monomethylsiloxan als Monomer wird dabei eine gewisse dreidimensionale, die Kohäsion verbessernde, Vernetzung erreicht.

In weiteren erfindungsgemäßen Polysiloxanen können die Methylgruppen ganz oder teilweise durch andere Alkyl- bzw. Phenylreste ersetzt sein.

Das Molekulargewicht und der Grad der Quervernetzung sind wichtige Parameter, die die Kohäsion und die Klebrigkeit des Polymers nach Entfernung des Lösemittels bestimmen.

Auf Grund der freien OH-Gruppen neigen Polysiloxane gemäß obiger Strukturformel in Gegenwart von basischen Substanzen, z. B. Aminen, zu Kondensationsreaktionen, die im Laufe der Zeit die Klebrigkeit reduzieren. In einer besonderen aminresistenten Form dieser Polymere sind deshalb alle freien Si-OH Gruppen in Si-O- C $(CH_3)_3$ Gruppen umgewandelt.

Generell zeichnen sich Polysiloxane durch eine gute Hautverträglichkeit, eine geringes Lösevermögen für die meisten Wirkstoffe und einen extrem hohen Diffusionskoeffizienten für den gelösten Anteil des Wirkstoffs aus. Im Sinne dieser Erfindung ist speziell die geringe Lösekapazität und der hohe Diffusionskoeffizient von besonderer Bedeutung.

Wegen der geringen Lösekapazität ist selbst bei einer geringen Wirkstoffbeladung der größte Teil des Wirkstoffs ungelöst. Durch geeignete Wahl des Verhältnisses von Wirkstoff zu Polysiloxan kann in jedem Fall dafür gesorgt werden, daß bei einer Löslichkeit des Wirkstoffs von nicht mehr als 5 Gew. % in der Matrix mindestens 80 % des Wirkstoffs ungelöst vorliegen. Da deshalb nur ein kleiner Teil der während der Applikationszeit abzugebenden Menge an Wirkstoff in gelöster Form in der Matrix vorliegt, ist das Nachlösen des Wirkstoffs und seine schnelle Diffusion zur Abgabefläche wichtig, um die Konzentration des gelösten Wirkstoffs in der Matrix in der Nähe der Sättigungskonzentration zu halten und damit eine konstant hohe thermodynamische Aktivität des Wirkstoffs über den Applikationszeitraum zu gewährleisten. Polysiloxane erfüllen auch diese Bedingung in idealer Weise auf Grund ihrer hohen Diffusibilität.

Bei der externen Anwendung von Wärme wird das TTS und damit die Matrix auf Temperaturen bis zu 50 °C erwärmt. Es ist dabei ausnahmslos davon auszugehen, daß aus der Temperaturerhöhung eine Erhöhung der Sättigungslöslichkeit des Wirkstoffs und eine Erhöhung der Konzentration des gelösten Wirkstoffs resultiert. Da sich dadurch die thermodynamische Aktivität des Wirkstoffs in der Matrix und der Diffusionskoeffizient des Wirkstoffs im Stratum Corneum erhöht, steigt die Abgabegeschwindigkeit des Wirkstoffs aus dem TTS an den Organismus.

Da bei Rückkehr zu Normalbedingungen (d. h.: Hauttemperatur) die Menge an gelöstem Wirkstoff zunächst unverändert bleibt, ist die Matrix wegen der nun wieder erniedrigten Sättigungslöslichkeit übersättigt. Da dieser Effekt zu einer nun bei Hauttemperatur ebenfalls erhöhten thermodynamischen Aktivität und damit einem erhöhten Wirkstoffflux durch die Haut führt, ist es wichtig, dass die Übersättigung schnell abgebaut wird. Polysiloxane als Grundpolymer für Matrices erfüllen diese Bedingung wegen ihrer schlechten Löslichkeit für Wirkstoffe in idealer Weise, da auf Grund der niedrigen Sättigungslöslichkeit von Wirkstoffen in solchen Matrices die Rückkehr der Konzentration des angelösten Wirkstoffes zur Sättigungskonzentration durch die Wirkstoffabgabe an den Organismus innerhalb kurzer Zeit erreicht

wird. Eine Rückkehr zur Sättigungslöslichkeit durch eine Rekristallisation des über der Sättigungslöslichkeit gelösten Anteils Wirkstoffs ist nur theoretisch möglich, da in Polymeren Wirkstoffe bzw. andere gelöste Substanzen nur schwer bzw. sehr langsam auskristallisieren.

Der oben geschilderte Sachverhalt kann durch eine Modellrechnung an 2 typischen TTS verdeutlicht werden. Dabei wird ein TTS, das den ungünstigsten Fall eines TTS auf Basis eines Silikonklebers im Sinne dieser Erfindung darstellt mit einem durchaus typisches TTS auf Basis von Polyacrylatklebern verglichen.

**Annahmen**:

| | |
|---|---|
| TTS Fläche: | 10 $cm^2$ |
| Wirkstoffbeladung: | 20 mg/ TTS |
| Sättigungslöslichkeit in Sililconsystem: | 5 % (g/g) |
| Beschichtungsgewicht Silikonsystem: | 80 $g/m^2$ |
| Sättigungslöslichkeit in Polyacrylatsystem: | 20 % |
| Beschichtungsgewicht Polyacrylatsystem: | 50 g/ $m^2$ |
| Wirkstoffabgabe: | 50 µg/ h |
| Temperaturerhöhung: | 32 °C → 40 °C |
| Erhöhung der Sättigungslöslichkeit bei 40 °C: | 10 % |

| | **Silikon-TTS** | **Polyacrylat-TTS** |
|---|---|---|
| Gewicht der Matrix | 16 mg | 10 mg |
| Wirkstoffbeladung | 4 mg | 4 mg |
| davon gelöst bei 32 °C | 0,8 mg [1)] | 2(mg [2)] |
| davon gelöst bei 40 °C | 0,88 mg | 2,2 mg |
| Zeit bis zur Rückkehr zur Sättigungslöslichkeit bei 32 °C [3)] | 1,6 Stunden | 4,0 Stunden |

1) Sättigungslöslichkeit 5 Gew. %; entspricht 20 % der Gesamtwirkstoffmenge
2) Sättigungslöslichkeit 20 Gew. %, entspricht 50 % der Gesamtwirkstoffmenge
3) Berechnung:

$$\frac{(\text{mg Wirkstoff gelöst bei } 40\,°C - \text{mg Wirkstoff gelöst bei } 32\,°C) * 1000}{\text{Abgaberate } [\,\mu g/\,h\,]}$$

Etwas vereinfacht wurde dafür angenommen, daß die Wirkstoffabgabe unmittelbar nach Ende der Wärmeanwendung wieder 50 µg/ h Stunde beträgt.

Qualitative sind die Unterschiede im Verhalten von beiden TTS in folgender Zeichnung verdeutlicht.

Figur 1: Verhalten von Silikon- und Polyacrylat-TTS nach Anwendung von Wärme zur Steigerung der Permeationsrate

Bei einem TTS, das schon anfänglich den Wirkstoff unterhalb der Sättigungskonzentration enthält, kann sich kein Wirkstoff nachlösen, wenn infolge der Temperaturerhöhung die Sättigungslöslichkeit steigt und die Differenz zwischen tatsächlicher Konzentration und Sättigungskonzentration größer wird. Dadurch fällt die Erhöhung der Permeationsrate im Vergleich zu gesättigten Systemen schwächer aus, wobei die Erhöhung des Diffusionskoeffizienten im Stratum Corneum den wesentlichen Beitrag leistet.

Wenn bei solchen Systemen die Permeationsrate durch Erwärmung erhöht wird, wird durch die verstärkte Wirkstoffabgabe während der Wärmeanwendung die Wirkstoffkonzentration schneller erniedrigt. Bei Rückkehr zu Normalbedingungen bedeutet dies, daß die Wirkstoffabgabe in Abhängigkeit von der zusätzlich abgegebenen Wirkstoffmenge erniedrigt ist. Damit sind solche untersättigten Systeme weitgehend unbrauchbar in Verbindung mit Wärmeanwendungen zur Steigerung der Permeationsraten, wenn eine konstante Wirkstoffabgabe über den Applikationszeitraum zwischen den Wärmeanwendungsphasen gefordert ist. Lediglich Systeme mit einer hohen Wirkstoffbeladung und einer geringen Wirkstoffabgabe sind unter dieser Bedingung geeignet, da sich unter diesen Bedingungen die Wirkstoffkonzentration und damit die Abgaberate während der Applikationszeit bzw. durch die gesteigerte Wirkstoffabgabe während einer Hitzeanwendung relativ wenig ändert.

Erkauft wird sich dies allerdings dann mit einer geringeren Wirkstoffausnutzung und bei teuren Wirkstoffen sind solche Systeme unwirtschaftlich.

TTS im Sinne dieser Erfindung können hergestellt werden, indem der Wirkstoff in der Lösung des Polysiloxans suspendiert wird, alle anderen Hilfsstoffe zugegeben werden und die Masse durch Rühren homogenisiert wird. Nach dem Beschichten auf eine geeignete Folie wird das Lösemittel des Klebers abgedampft und der getrocknete Film mit der Rückschicht des TTS kaschiert. Aus diesem Laminat werden dann die fertigen TTS gestanzt. Die gesamte Prozedur wird an Beispiel 1 mit Fentanyl als Base exemplarisch dargestellt. In Beispiel 2 wird die Herstellung eines TTS mit einer zusätzliche Steuermembran beschrieben.

Als Wirkstoffe, die für das erfindungsgemäße transdermales therapeutische System infrage kommen, seien die folgenden genannt:

Analgetika, wie z. B. Fentanyl oder eine Fentanyl-analoge Substanz, Butorphanol, Oxicodon, Ketorolac, Buprenorphin, Morphin, Tetracain, Lidocain, Bupivacain, Prilocain und Benzocain;
Antiparkinson-Medikamente wie z. B.Biperiden, Selegillin, Pramipexol
Broncholytika wie z. B. Salbutamol, Tulobuterol
Antiepileptika wie z. B. Clonazepam, Tiagabin

**Beispiel 1: TTS mit Fentanyl als Wirkstoff ohne Steuermembran**

3 g Fentanyl Base werden zu 138 g einer 70 prozentigen Lösung eines Silikonklebers in n-Heptan ( BIO-PSA 4301, Dow Corning ) gegeben und durch Rühren in dieser Lösung suspendiert. Die Suspension wird dann auf eine geeignete wiederentfernbare Schutzfolie ( Scotchpak 1022, 3M ) in einer Dicke beschichtet, daß nach dem Entfernen des Lösemittels ein Beschichtungsgewicht von 100 g/ $m^2$ resultiert. Der getrocknete Film wird nun mit einer 23 μm dicken Polyesterfolie, der Rückschicht des TTS, kaschiert und aus diesem Laminat die TTS gestanzt. Die einzelnen TTS werden in einem Beutel aus einem heißsiegelbaren Packstofflaminat verpackt.

**Beispiel 2: TTS mit Fentanyl als Wirkstoff mit Steuermembran**

**a. Herstellung der Reservoirschicht**

3 g Fentanyl Base werden zu 138 g einer 70 prozentigen Lösung eines Silikonklebers in n-Heptan ( BIO-PSA 4301, Dow Corning) gegeben und durch Rühren in dieser Lösung suspendiert. Die Suspension wird dann auf eine geeignete wiederentfernbare Schutzfolie ( Scotchpak 1022, 3M ) in einer Dicke beschichtet, daß nach dem Entfernen des Lösemittels ein Beschichtungsgewicht von 100 g/ $m^2$ resultiert. Der getrocknete Film wird nun mit einer 23 μm dicken Polyesterfolie, der Rückschicht des TTS, kaschiert.

**b. Herstellung der Hautkleberschicht**

Die Lösung eines Silikonklebers mit einem Feststoffgehalt von 70 % in n-Heptan ( BIO-PSA 4301, Dow Corning) wird auf eine geeignete wiederentfernbare Schutzfolie ( Scotchpak

1022, 3M ) in einer Dicke beschichtet, daß nach dem Entfernen des Lösemittels ein Beschichtungsgewicht von 30 g/ $m^2$ resultiert. Auf den getrockneten Film wird nun die Steuermembran ( 50 μm dicke EVA-Film mit einem Vinylacetagehalt von 19 %) aufkaschiert.

**c. Herstellung des Gesamtlaminats**

Von der unter a hergestellten Reservoirschicht wird die wiederentfernbare Schutzfolie abgezogen und auf die Membran der unter b hergestellten Hautkleberschicht aufkaschiert. Aus dem Gesamtlaminat werden die Einzel-TTS gestanzt und in einem Beutel aus einem heißsiegelbaren Packstofflaminat verpackt.

**Patentansprüche**

1. Transdermales Therapeutisches System (TTS) für eine zeitweise erhöhte Abgaberate mindestens eines pharmakologischen Wirkstoffes durch Erwärmung des TTS auf eine Temperatur zwischen Hauttemperatur und 50°C einschließlich und Abkühlenlassen des TTS nach einer bestimmten Zeit auf Hauttemperatur, umfassend mindestens eine wirkstoffundurchlässige Rückschicht, mindestens eine Matrixschicht auf der Basis von mindestens einem Polysiloxan enthaltend den oder die oben genannten pharmakologischen Wirkstoffe, eine abziehbare Schutzschicht sowie ein internes und/oder ein externes wärmeabgebendes Element, wobei (a) die wirkstoffhaltige Matrixschicht ohne Berücksichtigung des Wirkstoffs zu mindestens 80 Gew.-% aus mindestens einem Polysiloxan besteht, (b) der Wirkstoff zu höchstens 20 Gew.-% in gelöster Form vorliegt und (c) der Wirkstoff in diesem Polysiloxan eine Sättigungslöslichkeit von höchstens 5 Gew.-% besitzt,
**dadurch gekennzeichnet, dass** nach Beendigung der Erwärmung des TTS die thermodynamische Aktivität des Wirkstoffs in der Matrixschicht schnell auf das Ausgangsniveau zurückgeht.

2. TTS gemäß Anspruch 1, **dadurch gekennzeichnet, dass** das Polysiloxan ein Polydialkylsiloxan ist, worin eine oder mehrere Alkylgruppen durch Phenylreste ersetzt sein können.

3. TTS gemäß einem oder mehreren der vorhergehenden Ansprüche, **dadurch gekennzeichnet, dass** das Polysiloxan aminresistent ist.

4. TTS gemäß einem oder mehreren der vorhergehenden Ansprüche, **dadurch gekennzeichnet, dass** das Polysiloxan selbstklebende Eigenschaften hat.

5. TTS gemäß einem oder mehreren der vorhergehenden Ansprüche, **dadurch gekennzeichnet**, das das Polysiloxan ein Polydimethylsiloxan ist.

6. TTS gemäß einem oder mehreren der vorhergehenden Ansprüche, **dadurch gekennzeichnet, dass** die wirkstoffhaltige Schicht hautseitig mit einer Steuermembran und diese optional mit einer Kleberschicht zur Verankerung auf der Haut versehen ist.

7. TTS gemäß Anspruch 8, **dadurch gekennzeichnet, dass** die Kleberschicht zur Verankerung auf der Haut ebenfalls mit Wirkstoff beladen ist.

8. TTS gemäß einem oder mehreren der vorhergehenden Ansprüche, **dadurch gekennzeichnet, dass** die wirkstoffhaltige Schicht einen die Barriereeigenschaften der Haut reduzierenden Hilfsstoff enthalten.

9. TTS gemäß Anspruch 10, **dadurch gekennzeichnet, dass** der die Barriereeigenschaften der Haut reduzierende Hilfsstoff Ölsäure ist.

10. TTS gemäß einem oder mehreren der vorhergehenden Ansprüche, **dadurch gekennzeichnet, dass** der Wirkstoff ein topisch oder systemisch wirksames Analgetikum ist.

11. TTS gemäß Anspruch 12, **dadurch gekennzeichnet, dass** der Wirkstoff Fentanyl oder eine fentanyl-analoge Substanz, Butorphanol, Oxycodon, Ketorolac, Buprenorphin, Morphin, Tetracain, Lidocain, Bubivacain, Prilocain oder Benzocain ist.

12. TTS gemäß einem oder mehreren der vorhergehenden Ansprüche, **dadurch gekennzeichnet**, das der Wirkstoff ein Antiparkinsonmittel, ein Impfserum, ein Broncholytikum, ein Antiepileptikum oder ein Suchtentwöhnungsmittel ist.

13. TTS gemäß Anspruch 11, **dadurch gekennzeichnet, dass** der Wirkstoff Fentanyl und/oder eine Fentanylanaloge Substanz ist.

14. Verpackungseinheit, **dadurch gekennzeichnet, dass** sie einzeln verpackt

    a) ein TTS gemäß Anspruch 1, jedoch ohne das interne und ohne das externe wärmeabgebende Element, und
    b) ein oder mehrere externe wärmeabgebende Elemente enthält.

## Claims

1. A transdermal therapeutic system (TTS) for a temporarily raised release rate of at least one pharmacological active substance by heating the TTS to a temperature between the skin temperature and 50°C inclusive and allowing the TTS to cool down after a certain time to skin temperature, comprising at least one backing layer impermeable to active substance, at least one matrix layer based on at least one polysiloxane including the abovementioned pharmacological active substance or substances, a detachable protective layer, and an internal and/or external heating element, where (a) the active substance-containing layer comprises, not taking the active substance into account, at least 80% by weight of a polysiloxane, (b) not more than 20% by weight of active substance is present in dissolved form, and (c) the active substance has a saturation solubility of 5% by weight in this polysiloxane.

2. The TTS as claimed in claim 1, **characterized in that** the polysiloxane is a polydialkylsiloxane wherein one or more alkyl groups may be replaced by phenyl radicals.

3. The TTS as claimed in one or more of the preceding claims, **characterized in that** the polysiloxane is amine-resistant.

4. The TTS as claimed in one or more of the preceding claims, **characterized in that** the polysiloxane has self-adhesive properties.

5. The TTS as claimed in at least one of the preceding claims, **characterized in that** the polysiloxane is a polydimethylsiloxane.

6. The TTS as claimed in one or more of the preceding claims, **characterized in that** the active substance-containing layer is provided on the skin side with a control membrane, and the latter is optionally provided with an adhesive layer for affixing to the skin.

7. The TTS as claimed in claim 8, **characterized in that** the adhesive layer for affixing to the skin is likewise loaded with active substance.

8. The TTS as claimed in one or more of the preceding claims, **characterized in that** the active substance-containing layer comprise an excipient which reduces the barrier properties of the skin.

9. The TTS as claimed in claim 10, **characterized in that** the excipients which reduce the barrier properties of the skin is oleic acid.

10. The TTS as claimed in one or more of the preceding claims, **characterized in that** the active substance is an analgesic having topical or systemic activity.

11. The TTS as claimed in claim 12, **characterized in that** the active substance is fentanyl or a fentanyl-analogous substance, butorphanol, oxycodone, ketorolac, buprenorphine, morphine, tetracaine, lidocaine, bupivacaine, prilocaine or benzocaine.

12. The TTS as claimed in at least one preceding claims, **characterized in that** the active substance is an antiparkinson agent, a vaccine, a bronchospasmolytic, an antiepileptic or an anticraving agent.

13. The TTS as claimed in claim 11, **characterized in that** the active substance is fentanyl and/or a fentanyl-analogous substance.

14. A packaging unit, **characterized in that** it comprises, packaged individually,

   a) a TTS as claimed in claim 1, but without the internal and without the external heating element, and
   b) one or more external heating elements (heatpacks).

## Revendications

1. Système thérapeutique transdermique (TTS) pour une délivrance à taux temporairement augmenté d'au moins un principe actif pharmacologique par réchauffage du TTS à une température comprise entre la température de la

peau et 50°C compris, et par refroidissement du TTS après une durée déterminée à la température de la peau, comprenant au moins une couche arrière imperméable au principe actif, au moins une couche matricielle à base d'au moins un polysiloxane renfermant le ou lés principes actifs pharmacologiques cités ci-dessus, une couche protectrice amovible, ainsi qu'un élément interne et/ou externe fournissant la chaleur,

(a) la couche matricielle renfermant le principe actif est constituée, sans la prise en compte du principe actif, par au moins 80 % en masse d'un polysiloxane,
(b) le principe actif se présente sous forme dissoute à 20 % en masse au maximum, et
(c) le principe actif dans ce polysiloxane possède une solubilité de saturation d'au maximum 5 %,

**caractérisé en ce que**, après avoir terminé le réchauffage du TTS, l'activité thermodynamique du principe actif dans la couche matricielle revient rapidement au niveau de départ.

**2.** TTS selon la revendication 1, **caractérisé en ce que** le polysiloxane est un polydialkylsiloxane, dans lequel un ou plusieurs groupes alkyle peuvent être remplacés par des restes phényle.

**3.** TTS selon l'une des revendications précédentes, **caractérisé en ce que** le polysiloxane est résistant aux amines.

**4.** TTS selon une ou plusieurs des revendications précédentes, **caractérisé en ce que** le polysiloxane possède des propriétés autocollantes.

**5.** TTS selon une ou plusieurs des revendications précédentes, **caractérisé en ce que** le polysiloxane est un polydiméthylsiloxane.

**6.** TTS selon une ou plusieurs des revendications précédentes, **caractérisé en ce que** la couche renfermant le principe actif est munie du côté de contact avec la peau d'une membrane de contrôle et celle-ci est facultativement pourvue d'une couche adhésive afin qu'elle soit fixée à la peau.

**7.** TTS selon la revendication 8, **caractérisé en ce que** la couche adhésive prévue pour la fixation à la peau est également chargée du principe actif.

**8.** TTS selon une ou plusieurs des revendications précédentes, **caractérisé en ce que** la couche renfermant le principe actif renferme une matière auxiliaire réduisant les propriétés barrière de la peau.

**9.** TTS selon la revendication 10, **caractérisé en ce que** la matière auxiliaire réduisant les propriétés barrière de la peau est l'acide oléique.

**10.** TTS selon une ou plusieurs des revendications précédentes, **caractérisé en ce que** le principe actif est un analgésique à efficacité topique ou systémique.

**11.** TTS selon la revendication 12, **caractérisé en ce que** le principe actif est le fentanyle ou une substance analogue au fentanyle, le butorphanol, l'oxycodone, le kétorolac, la buprénorphine, la morphine, la tétracaïne, la lidocaïne, la bupivacaïne, la prilocaïne ou la benzocaïne.

**12.** TTS selon une ou plusieurs des revendications précédentes, **caractérisé en ce que** le principe actif est un agent anti-parkinsonien, un sérum de vaccination, un broncholytique, un antiépileptique ou un agent antidépendance.

**13.** TTS selon la revendication 11, **caractérisé en ce que** le principe actif est le fentanyle et/ou une substance analogue au fentanyle.

**14.** Unité d'emballage, **caractérisée en ce qu'**elle emballe individuellement

a) un TTS selon la revendication 1 mais sans l'élément interne et sans l'élément externe fournissant la chaleur, et
b) un ou plusieurs éléments externes fournissant la chaleur.